Europäisches Patentamt

European Patent Office

Office européen des brevets

(19)

(11) **EP 0 729 935 B1**

(12) **FASCICULE DE BREVET EUROPEEN**

(45) Date de publication et mention
de la délivrance du brevet:
**09.06.1999 Bulletin 1999/23**

(51) Int. Cl.⁶: **C07C 53/19**, C07C 51/363

(21) Numéro de dépôt: **96400442.8**

(22) Date de dépôt: **01.03.1996**

(54) **Procédé de préparation de l'acide 3-chloropropionique**

Verfahren zur Herstellung von 3-Chlorpropionsäure

Process for preparing 3-chloropropionic acid

(84) Etats contractants désignés:
**AT BE CH DE DK ES FI FR GB GR IE IT LI LU NL
PT SE**

(30) Priorité: **03.03.1995 FR 9502484**

(43) Date de publication de la demande:
**04.09.1996 Bulletin 1996/36**

(73) Titulaire: **ELF ATOCHEM S.A.
92800 Puteaux, Hauts-de-Seine (FR)**

(72) Inventeurs:
 • **Drivon, Gilles
 69850 Saint Martin en Haut (FR)**
 • **Ruppin, Christophe
 69310 Pierre Benite (FR)**

(56) Documents cités:
 **FR-A- 2 009 238            US-A- 2 571 901**

## Description

La présente invention concerne un procédé de préparation de l'acide 3-chloropropionique par hydrochloration de l'acide acrylique.

L'acide 3-chloropropionique est un intermédiaire important dans la fabrication des biocides, des colorants, des produits pharmaceutiques, des produits cosmétiques et des additifs pour les matières plastiques.

Le brevet russe SU 438639 décrit un procédé qui consiste à faire réagir de l'acide acrylique avec une solution aqueuse d'acide chlorhydrique en présence d'un sel d'ammonium quaternaire.

Le mélange réactionnel est maintenu à une température voisine de l'ambiante sous forte agitation pendant plusieurs heures, puis, l'eau et l'acide chlorhydrique non réagis sont éliminés par distillation sous pression réduite.

Après séchage azéotropique, l'acide 3-chloropropionique est obtenu avec un rendement de 87 %.

Le brevet américain US 2759018 décrit un procédé de préparation d'acide 3-chloropropionique par réaction d'une solution aqueuse d'acide acrylique avec de l'acide chlorhydrique gazeux à une température inférieure à 60° C. La concentration pondérale en acide acrylique de la solution aqueuse d'acide acrylique est de préférence comprise entre 10 % et 30 %.

Les conditions opératoires utilisées dans les deux procédés précédemment mentionnés sont telles que la réaction d'hydrochloration de l'acide acrylique est très lente et les rendements en acide 3-chloropropionique sont faibles. En outre à la fin de l'opération, la séparation nécessaire de l'eau ainsi que des quantités non négligeables d'acide chlorhydrique et/ou de sel et ammonium quaternaire sont des opérations coûteuses et rédhibitoires pour l'économie d'un procédé industriel.

Le brevet allemand DE 2555043 décrit un procédé de fabrication d'acide 3-chloropropionique qui consiste à faire réagir sous des pressions allant de 1,5 bars à 5 bars, de l'acide acrylique avec de l'acide chlorhydrique gazeux selon un rapport molaire acide acrylique / acide chlorhydrique allant de 1 à 1,5 à une température comprise entre 40° C et 80° C.

Bien que ce procédé conduise à une forte productivité alliée à une bonne sélectivité, il présente l'inconvénient majeur d'opérer sous pression d'acide chlorhydrique gazeux ce qui nécessite l'utilisation d'un appareillage très spécifique et coûteux.

On a maintenant trouvé un procédé semi-continu de préparation de l'acide 3-chloropropionique par hydrochloration d'acide acrylique, caractérisé en ce que, dans un pied de cuve, constitué essentiellement par :

- 70 % à 100 % en poids d'acide 3-chloropropionique et,
- 0 % à 30 % en poids d'eau ou d'une solution aqueuse d'acide chlorhydrique, on introduit simultanément, de l'acide chlorhydrique gazeux et de l'acide acrylique, puis éventuellement on continue l'introduction de l'acide chlorhydrique gazeux.

Selon la présente invention, on introduit simultanément l'acide chlorhydrique et l'acide acrylique, selon un rapport molaire acide chlorhydrique / acide acrylique compris entre 0,70 et 1,30 et, de préférence selon un rapport molaire compris entre 0,80 et 1,15.

Selon la présente invention, le pied de cuve est constitué par de l'acide 3-chloropropionique pur, ou par de l'acide 3-chloropropionique dilué par une quantité pondérale d'eau comprise, de préférence entre 5 % et 20 %, ou bien encore par de l'acide 3-chloropropionique dilué par une quantité pondérale d'une solution aqueuse d'acide chlorhydrique comprise de préférence entre 5 % et 25 %.

La concentration pondérale en acide chlorhydrique des solutions aqueuses d'acide chlorhydrique utilisables selon la présente invention est au moins égale à 20 % et, de préférence comprise entre 22 % et 37 %.

Le pied de cuve est préalablement chauffé à une température au moins égale à 30° C et, de préférence, à une température comprise entre 40° C et 60°. Pendant l'introduction simultanée de l'acide chlorhydrique gazeux et de l'acide acrylique, pendant la poursuite éventuelle de l'introduction de HClgaz et après la fin d'introduction des réactifs, la température du milieu réactionnel est avantageusement maintenue entre 40°C et 60°C.

Selon la présente invention, on opère à pression atmosphérique.

Selon la présente invention, le procédé est mis en oeuvre dans un réacteur en acier vitrifié ou en verre muni d'une agitation, de moyens de chauffage, d'introductions de gaz et/ou de liquide, d'une prise de température, d'une colonne de distillation éventuellement reliées à un système de piégeage de HCl.

L'agitation du milieu réactionnel peut être réalisée par tout moyen assurant un bon contact gaz/liquide.

L'acide acrylique utilisable selon la présente invention a une pureté au moins égale à 95 % et est généralement stabilisé au moyen de faibles quantités d'alkylesters de l'hydroquinone. L'acide acrylique peut également contenir des impuretés inertes en très faibles quantités tels que acide acétique, acide propionique.

Selon la présente invention, le pied de cuve représente au plus 30 % en volume du réacteur utilisé pour la mise en oeuvre du procédé. Le gaz chlorhydrique peut être introduit au moyen d'un tube plongeant dans ledit pied de cuve.

Il peut être également introduit par tout dispositif approprié par le fond du réacteur.

Selon la présente invention, les quantités d'acide chlorhydrique gazeux et d'acide acrylique introduites simultanément sont proches des quantités stoechiométriques de la réaction :

$$HCl + CH_2 = CH - COOH \rightarrow Cl\, CH_2 - CH_2 - COOH$$

Ces quantités sont telles que le rapport molaire acide chlorhydrique gazeux / acide acrylique est compris entre 0,70 et 1,30 et de préférence entre 0,80 et 1,15.

Dans l'éventualité où l'on opère avec un léger excès d'acide chlorhydrique par rapport à l'acide acrylique on minimise la concentration en acide acrylique dans le milieu réactionnel.

Cependant, cette façon d'opérer peut entraîner de faibles pertes d'acide chlorhydrique dans les évents.

Dans le cas où l'on opère avec un léger défaut d'acide chlorhydrique par rapport à l'acide acrylique les pertes éventuelles en acide chlorhydrique dans les évents sont minimisées mais la concentration en acide acrylique résiduelle dans le milieu réactionnel est plus élevée.

Selon la présente invention, l'introduction du gaz chlorhydrique consécutive à l'introduction simultanée d'HClgaz et d'acide acrylique peut être maintenue pendant une durée suffisante de façon à obtenir notamment une quantité pondérale résiduelle en acide acrylique inférieure à 2 % et de préférence inférieure ou égale à 1 %.

Cette durée d'introduction d'acide chlorhydrique gazeux peut varier dans une large mesure et est fonction, notamment des quantités d'acide chlorhydrique gazeux introduites simultanément avec l'acide acrylique, des quantités d'eau contenues dans le pied de cuve et de l'efficacité du contact gaz/liquide réalisé par le système d'agitation du milieu réactionnel.

Elle est au plus égale à 10 heures et, de préférence , comprise entre 1 et 5 heures

Cette introduction d'acide chlorhydrique gazeux, consécutive à l'introduction simultanée de HClgaz et d'acide acrylique, peut être supprimée dans le cas ou le pied de cuve est constitué par de l'acide 3-chloropropionique dilué par une solution aqueuse d'acide chlorhydrique.

L'état d'avancement de la réaction peut être suivi soit par détermination de l'acide 3-chloropropionique formé à l'aide d'une analyse chromatographique en phase gazeuse et / ou d'une analyse par résonance magnétique nucléaire du proton, soit par mesure de la quantité d'acide chlorhydrique par dosage potentiométrique.

Selon la présente invention, dans le cas ou l'on opère avec un pied de cuve exclusivement constitue d'acide 3-chloropropionique et lorsque l'introduction d'acide chlorhydrique gazeux consécutive à l'introduction simultanée de HClgaz et d'acide acrylique est terminée, on élimine l'acide chlorhydrique dissous non réagi, soit par dégazage à l'aide d'un gaz inerte tel que l'azote, soit par mise sous pression réduite.

Dans le cas ou l'on opère à partir d'un pied de cuve contenant de l'acide 3-chloropropionique dilué avec de l'eau ou avec une solution aqueuse d'acide chlorhydrique, on élimine l'acide chlorhydrique aqueux et/ou dissous par un ététage azéotropique sous pression réduite.

Dans tous les cas on récupère l'acide 3-chloropropionique avec un rendement quasi quantitatif par rapport à l'acide acrylique mis en oeuvre et un titre en acide 3-chloropropionique au moins égal à 94 %.

L'acide 3-chloropropionique ainsi obtenu peut ensuite être directement employé comme réactif intermédiaire dans la synthèse de produits pharmaceutiques.

Il peut également être dilué avec de l'eau de manière à obtenir des solutions acqueuses liquides facilement transportables sans risque de cristallisation.

Le procédé selon l'invention présente l'avantage d'être effectué à pression atmosphérique ne nécessitant pas d'appareil spécifique.

Selon la présente invention, on a également une cinétique de réaction rapide et un rendement pratiquement quantitatif en acide 3-chloropropionique par rapport à l'acide acrylique.

Le procédé selon l'invention présente également l'avantage de conduire à un minimum de pertes en acide chlorhydrique gazeux et à un acide 3-chloropropionique ayant une teneur pondérale en acide acrylique résiduelle inférieure à 2 %.

Les exemples qui suivent illustrent l'invention.

## Exemple 1

Dans un réacteur en verre de 1 l, on charge 217g d'acide 3-chloropropionique stabilisé avec 200 ppm d'ester méthylique de l'hydroquinone.

On chauffe vers 45° C puis on introduit simultanément en 5 h :

- 360 g d''acide acrylique à un débit de 1 mole/h
- 237 g d'HCl anhydre à un débit de 1,3 moles/h

tout en maintenant la température à une valeur d'environ 45°C.

Lorsque tout l'acide acrylique est introduit, la concentration en acide acrylique dans le milieu réactionnel est égale à 5,8 % on maintient alors l'alimentation d'HCl à un débit de 0,5 mole/h jusqu'à ce que la quantité théorique d'HCl soit absorbée, soit pendant 2 heures.

L'avancement de la réaction est suivi par dosage potentiométrique d'HCl absorbé.

Le milieu réactionnel est ensuite maintenu 1 h à environ 45°C puis dégazé pendant 1 h par un léger bullage d'azote.

On obtient 756 g d'un produit brut ayant une pureté RMN > 94 % contenant moins de 1 % d'acide acrylique résiduel et moins de 0,5 % d'acide chlorhydrique.

Ce produit est soit stocké tel quel, soit dilué par 185 g d'eau distillée de manière à obtenir une solution aqueuse à une concentration de 80 % en acide 3-chloropropionique, solution qui ne cristallise pas à une température supérieure à 10°C.

## Exemple 2, 3 et 4

On opère dans le même appareillage que dans l'exemple 1 en utilisant dans chaque exemple un pied de cuve constitué par 217 g d'acide 3-chloropropionique pur.

Le tableau unique ci-après rassemble les paramètres réactionnels ainsi que les concentrations en acide acrylique dans les milieux réactionnels.

Dans ce tableau on désigne par :

T (°C)        températures de chauffage du pied de cuve et du milieu réactionnel durant les étapes I et II,

ETAPE I       étape d'introduction simultanée dans le pied de cuve de l'acide acrylique et de l'acide chlorhydrique gazeux (HClgaz),

EPAPE II      introduction de HClgaz,

$D_{INT}$        durée d'introduction en heures,

$[AA]_I$         concentration pondérale en acide acrylique dans le milieu réactionnel après l'étape I,

Après l'étape II, le milieu réactionnel est maintenu pendant 1 heure à environ 45°C puis dégazé pendant 1 heure par un léger bullage d'azote.

On obtient ainsi un acide 3-chloropropionique dont la concentration pondérale en acide acrylique résiduel est désignée par $[AA]_{II}$ dans le tableau unique.

TABLEAU UNIQUE

| EXEMPLE | T (°C) | ETAPE I | | | | ETAPE II | | $[AA]_{II}$ (%) |
|---|---|---|---|---|---|---|---|---|
| | | $D_{INT}$ (h) | Débit (mole/h) | $[AA]_I$ (%) | HClgaz | | | |
| Acide Acrylique | HClgaz | $D_{INT}$ (h) | Débit (mole/h) | | | | | |
| 2 | 45-50 | 5 | 1 | 1,15 | 10,7 | 3 | 0,5 | ≤ 1 |
| 3 | 45-50 | 5 | 1 | 1 | 15,8 | 4 | 0,5 | ≤ 1 |
| 4 | 45-50 | 5 | 1 | 0,8 | 22,5 | 5 | 0,5 | ≤ 1 |

## Exemple 5

On opère dans le même appareillage que dans l'exemple 1.

On charge initialement 217 g d'acide 3-chloropropionique pur et 19 g d'eau ce qui correspond à un pied de cuve contenant 8 % en poids d'eau, puis on introduit simultanément pendant 5 heures, à une température de 45°C-50°C, de l'acide chlorhydrique gazeux et de l'acide acrylique à un même débit de 1 mole/heure.

A ce stade, la concentration pondérale en acide acrylique dans le milieu réactionnel est de 5,2 %.

On poursuit ensuite pendant 2 heures l'addition de HCl gazeux à un débit de 0,5 mole/heure.

Le milieu réactionnel est ensuite maintenu une heure à environ 45°C puis étêté sous pression réduite pour éliminer HCl aqueux.

On obtient 752 g d'acide 3-chloropropionique contenant moins de 1 % d'acide acrylique résiduel.

<u>Exemple 6</u>

On opère dans le même appareillage que dans l'exemple 1.

On charge initialement 217 g d'acide 3-chloropropionique pur et 84 g de solution aqueuse d'acide chlorhydrique à 33 %.

On introduit ensuite, simultanément et pendant 5 heures, à une température comprise entre 45°C et 50°C, de l'acide chlorhydrique gazeux à un débit de 1,1 moles/heure et de l'acide acrylique à un débit de 1 mole/heure.

Le milieu réactionnel est ensuite maintenu pendant 1 heure à environ 45°C puis on effectue un étetage azéotropique sous pression réduite, pour éliminer l'acide chlorhydrique aqueux.

On récupère 745 g d'acide 3-chloropropionique contenant moins de 1 % en poids d'acide acrylique résiduel.

L'acide chlorhydrique aqueux peut ensuite être réutilisé pour diluer l'acide 3-chloropropionique du pied de cuve d'une opération suivante.

## Revendications

1.   Procédé de préparation de l'acide 3-chloropropionique par hydrochloration d'acide acrylique, caractérisé en ce que, dans un pied de cuve, constitué essentiellement par :

   70 % à 100 % en poids d'acide 3-chloropropionique et,
   0 % à 30 en poids d'eau ou d'une solution aqueuse d'acide chlorhydrique, on introduit simultanément de l'acide chlorhydrique gazeux et de l'acide acrylique, que l'on opère à pression atmosphérique, puis, éventuellement on continue l'introduction de l'acide chlorhydrique gazeux

2.   Procédé selon la revendication 1, caractérisé en ce que le pied de cuve est constitué par de l'acide 3-chloropropionique.

3.   Procédé selon la revendication 1, caractérisé en ce que le pied de cuve est constitué par de l'acide 3-chloropropionique dilué par une quantité pondérale d'eau comprise entre 5 % et 20 %.

4.   Procédé selon la revendication 1, caractérisé en ce que le pied de cuve est constitué par de l'acide 3-chloropropionique dilué par une quantité pondérale d'une solution aqueuse d'acide chlorhydrique comprise entre 5 % et 25 %.

5.   Procédé selon l'une des revendications 1 à 4 caractérisé en ce que l'on introduit simultanément l'acide chlorhydrique gazeux et l'acide acrylique selon un rapport molaire HClgaz/acide acrylique compris entre 0,70 et 1,30.

6.   Procédé selon la revendication 5, caractérisé en ce que le rapport molaire HClgaz/acide acrylique est compris entre 0,80 et 1,15.

7.   Procédé selon l'une des revendications 1 à 6, caractérisé en ce que le pied de cuve est préalablement chauffé à une température au moins égale à 30°C et, de préférence, à une température comprise entre 40°C et 60°C.

## Claims

1.   Process for the preparation of 3-chloropropionic acid by hydrochlorination of acrylic acid, characterized in that hydrochloric acid gas and acrylic acid are simultaneously introduced into a sediment, essentially consisting of:

   70% to 100% by weight of 3-chloropropionic acid and,
   0% to 30% by weight of water or of an aqueous solution of hydrochloric acid, in that the procedure is carried out at atmospheric pressure, and then the introduction of the hydrochloric acid gas is optionally continued.

2.   Process according to claim 1, characterized in that the sediment consists of 3-chloropropionic acid.

3.   Process according to claim 1, characterized in that the sediment consists of 3-chloropropionic acid diluted with a quantity of water by weight of between 5% and 20%.

4.   Process according to claim 1, characterized in that the sediment consists of 3-chloropropionic acid diluted with a quantity of an aqueous hydrochloric acid solution by weight of between 5% and 25%.

5. Process according to one of claims 1 to 4, characterized in that hydrochloric acid gas and acrylic acid are simultaneously introduced in a HCl gas/acrylic acid mol ratio of between 0.70 and 1.30.

6. Process according to claim 5, characterized in that the HCl gas/acrylic acid mol ratio is between 0.80 and 1.15.

7. Process according to one of claims 1 to 6, characterized in that the sediment is heated beforehand to a temperature at least equal to 30°C and, preferably, to a temperature of between 40°C and 60°C.

**Patentansprüche**

1. Verfahren zur Herstellung von 3-Chlorpropionsäure durch Hydrochlorierung von Acrylsäure, dadurch gekennzeichnet, daß in einen Ansatz, der im wesentlichen aus

    70 bis 100 Gew.-% 3-Chlorpropionsäure und
    0 bis 30 Gew.-% Wasser oder einer wäßrigen Lösung von Chlorwasserstoff besteht, gleichzeitig gasförmiger Chlorwasserstoff und Acrylsäure eingeführt wird, daß unter Atmosphärendruck gearbeitet wird, und dann gegebenenfalls die Zugabe von gasförmigem Chlorwasserstoff fortgesetzt wird.

2. Verfahren nach Anspruch 1, dadurch gekennzeichnet, daß der Ansatz aus 3-Chlorpropionsäure besteht.

3. Verfahren nach Anspruch 1, dadurch gekennzeichnet, daß der Ansatz aus 3-Chlorpropionsäure besteht, die mit 5 bis 20 Gew.-% Wasser verdünnt ist.

4. Verfahren nach Anspruch 1, dadurch gekennzeichnet, daß der Ansatz aus 3-Chlorpropionsäure besteht, die mit 5 bis 25 Gew.-% einer wäßrigen Lösung von Chlorwasserstoff verdünnt ist.

5. Verfahren nach einem der Ansprüche 1 bis 4, dadurch gekennzeichnet, daß man gleichzeitig gasförmigen Chlorwasserstoff und Acrylsäure in einem molaren Verhältnis von HCl-Gas/Acrylsäure zwischen 0,70 und 1,30 einführt.

6. Verfahren nach Anspruch 5, dadurch gekennzeichnet, daß das molare Verhältnis HCl-Gas/Acrylsäure zwischen 0,80 und 1,15 beträgt.

7. Verfahren nach einem der Ansprüche 1 bis 6, dadurch gekennzeichnet, daß der Ansatz zuvor auf eine Temperatur von mindestens 30 °C, vorzugsweise auf eine Temperatur zwischen 40 °C und 60 °C erwärmt ist.